# EUROPEAN PATENT SPECIFICATION

(11) **EP 3 430 968 B1**
(45) Date of publication and mention of the grant of the patent: **14.07.2021**
(21) Application number: 16893914.8
(22) Date of filing: 17.03.2016
(51) Int. Cl.: A61B 1/005, A61B 34/00, A61B 34/30

(54) **FINE-TUNING MECHANISM OF ENDOSCOPE**
FEINABSTIMMUNGSMECHANISMUS EINES ENDOSKOPS
MÉCANISME DE RÉGLAGE FIN D'ENDOSCOPE

(43) Date of publication of application: 23.01.2019
(73) Proprietor: Shenzhen Yateks Optical Electronic Technology Co., Ltd., Shenzhen, Guangdong 518100 (CN)
(72) Inventor: YAN, Jiangfan, Shenzhen Guangdong 518100 (CN); ZHONG, Quan, Shenzhen Guangdong 518100 (CN); MA, Tianyue, Shenzhen Guangdong 518100 (CN); WANG, Zhongkai, Shenzhen Guangdong 518100 (CN)
(74) Representative: Dargiewicz, Joanna
(86) International application number: PCT/CN2016/076602
(87) International publication number: WO 2017/156749

(56) References cited:
- WO-A1-2014/109714
- CN-A- 102 122 068
- CN-A- 102 573 599
- CN-A- 102 770 060
- CN-B- 102 103 261
- CN-B- 102 711 582
- US-A- 4 203 430
- US-A- 4 483 326
- US-B1- 6 533 772

## Description

### TECHNICAL FIELD

The disclosure relates to an endoscope structure, and particularly to a precise fine-tuning mechanism for an endoscope.

### BACKGROUD OF THE PRESENT INVENTION

An endoscope is a multi-disciplinary universal tool, and has functions of detecting a deep point of a bent pipe, observing parts that cannot be directly viewed, observing an interior space structure and state in a sealed cavity and achieving long-distance observation and operation. With the rapid development of industrial technologies, instruments are increasingly used in all industries, and thus detection of the healthy status of the instrument becomes extremely important. Industrial detection gradually cuts a figure in industrial detection. The endoscope is an indispensable part in an industrial detection family.

In types of endoscopes, the endoscope capable of rotating in any direction is the most common one, and is achieved generally by adopting electric driving. An electric steering mechanism adopts a miniature steering engine which is pulled using four steel wires, is flexible to rotate and is precise to control. However, in the early mounting process and in the later long-term use process, four steel wire traction lines in the endoscope will appear loose and fatigued effects; due to influence of thermal expansion, steel wire traction lines and a corresponding traction mechanism have a loose phenomenon in the aspects of tensile load of a steel wire rope, a bending radius, a traction wheel material and a groove profile in the use of the steel wire rope, a material of the steel wire rope itself and use and maintenance of the steel wire rope such that the endoscope cannot precisely perform corresponding commands to result in a fact that an instrument cannot be used. Meanwhile, in the early instrument assembling process, the use of a fine-tuning device greatly reduces the complexity of a process procedure and the difficulty of the mounting, and thus it is extremely necessary to add the fine-tuning device in the use process of the instrument.

In the existing endoscope fine-turning technologies, a manner of gear meshing is generally used for locking and fine tuning. In the processing process of parts used in this structure, a structural design of internal gear teeth and external gear teeth of the parts is too complicated, and the processing costs of the internal teeth and the external teeth are relatively high due to tiny size.

Sherts Charles R et al. US6533772B1 discloses a guide wire torque device which has an elongate tubular body with interior long axial channel and a rotary clamp wheel for gripping and ungripping a guide wire extending through the channel. The clamp wheel is accommodated within an integral enlargement of the tubular body with a segment of the wheel surface extending through the surface of the enlargement for rotation by a user's thumb in order to grip and ungrip the guide wire for feeding, withdrawing or rotating the wire with respect to a catheter.

CN102103261 discloses an endoscope comprising a pull wire and a micro-mechanism for the pull wire, wherein the micro-mechanism comprises an adjusting lever, a runner, a first and second housing piece and a cover cylinder, wherein the cover cylinder is placed through the first housing piece and the runner inside the second housing piece, wherein the adjusting lever is placed inside the cover cylinder, wherein the cover cylinder has on its inside wall a holding section having cogs facing inside, wherein the rotating insert has cogs facing outside to be engaged with the holding section.

### SUMMARY OF PRESENT INVENTION

The present disclosure provides a novel fine-tuning mechanism for an endoscope.

The fine-tuning mechanism provided in the present disclosure includes: a rotating insert comprising a first positioning portion and a mounting portion for connecting a traction line; a wheel body on which the rotating insert is mounted in a rotatable manner, wherein the rotating insert is movable relative to the wheel body along a rotation center line of the rotating insert; a first position for locking the rotating insert and a second position for unlocking the rotating insert is provided along a moving path of the rotating insert; and the rotating insert is capable of being positioned at the first position; and a cover plate fixed relative to the wheel body, wherein the rotating insert is located between the cover plate and the wheel body; the cover plate comprises at least two second positioning portions arranged around the rotation center line of the rotating insert; when the wheel body moves to the first position, the first positioning portion forms a projection-receptacle engagement with one of the second positioning portions; and when the wheel body moves to the second position, the first positioning portion is disengaged from the second positioning portion.

As a further improvement of the fine-tuning mechanism, the rotating insert has a projected vertical end, and the wheel body has a wheel rotating hole into which the vertical end of the rotating insert is inserted; and the elastic member is arranged between a hole wall of the wheel rotating hole and the vertical end.

As a further improvement of the fine-tuning mechanism, the elastic member is connected with the rotating insert, and a restoring force of the rotating insert always drives the rotating insert to move toward the cover plate.

As a further improvement of the fine-tuning mechanism, the cover plate has a cover plate through hole, and the rotating insert has a boss exposed from the cover plate through hole, for operating the rotation of the rotating insert.

The fine-tuning mechanism includes a rotating insert, a wheel body and a cover plate. The rotating insert has a first positioning portion, is mounted on the wheel body in a rotatable manner and can move relative to the wheel body along a rotation center line of the rotating insert. The cover plate is fixed relative to the wheel body, and the cover plate has at least two second positioning portions arranged around the rotation center line of the rotating insert. When the wheel body moves to the first position, the first positioning portion forms a projection-receptacle engagement with one of the second positioning portions; and when the wheel body moves to the second position, the first positioning portion is disengaged from the second positioning portion. When fine tuning is desired, the rotating insert moves to the second position so that the first positioning portion is disengaged from the second positioning portion, then the rotating insert is rotated by a certain angle and then moves to the first position, so that the first positioning portion is matched with a next second positioning portion, thereby achieving fine tuning.

### DESCRIPTION OF THE DRAWINGS

Fig. 1 is a structural diagram of a fine-tuning mechanism for an endoscope according to the present disclosure;
Fig. 2 is a cross-sectional view of the fine-tuning mechanism shown in Fig. 1 in a disassembled state;
Fig. 3 is a structural diagram of a rotating insert according to an embodiment of the present disclosure;
Fig. 4 is a structural diagram of a rotating insert according to another embodiment of the present disclosure;
Fig. 5 is a structural diagram of a wheel body according to an embodiment of the present disclosure;
Fig. 6 is a cross-sectional view of the wheel body as shown in Fig. 5;
Fig. 7 is a structural diagram of a cover pate according to an embodiment of the present disclosure;
Fig. 8 is a structural diagram of a fine-tuning mechanism according to another embodiment of the present disclosure; and
Fig. 9 is a cross-sectional view of a wheel body according to an embodiment as shown in Fig. 8.

### DETAILED DESCRIPTION OF PREFERRED EMBODIMENTS

### Embodiment 1

This embodiment discloses a fine-tuning mechanism for an endoscope.

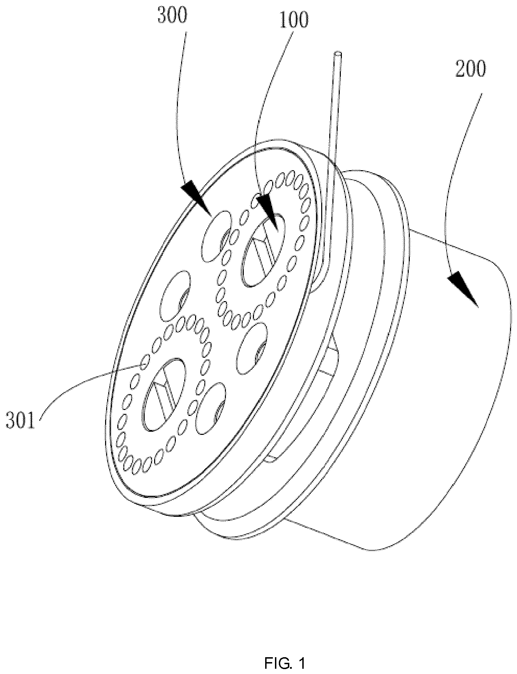

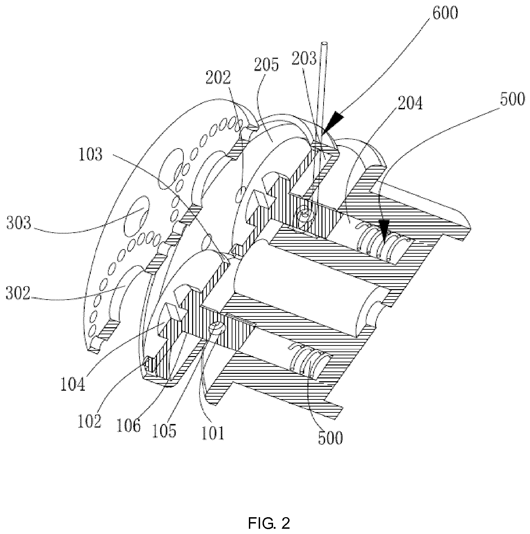

Referring to Figs. 1 and 2, the fine-tuning mechanism includes: a rotating insert 100 comprising a first positioning portion 102 and a mounting portion for connecting a traction line 600; a wheel body 200 on which the rotating insert 100 is mounted in a rotatable manner, and a cover plate 300 fixed relative to the wheel body 200.

The rotating insert 100 is movable relative to the wheel body 200 along a rotation center line of the rotating insert, a first position for locking the rotating insert 100 and a second position for unlocking the rotating insert 100 is provided along a moving path of the rotating insert, and the rotating insert 100 is capable of being positioned at the first position.

The rotating insert 100 is located between the cover plate 100 and the wheel body 200; the cover plate 300 comprises at least two second positioning portions 301 arranged around the rotation center line of the rotating insert 100; when the rotating insert 100 moves to the first position, the first positioning portion forms a projection-receptacle engagement with one of the second positioning portions 301; and when the rotating insert 100 moves to the second position, the first positioning portion 102 is disengaged from the second positioning portion 301.

When fine tuning is desired, the rotating insert 100 is moved to the second position so that the first positioning portion102 is disengaged from the second positioning portion 301, then the rotating insert 100 is rotated by a certain angle and then moves to the first position, so that the first positioning portion 102 is matched with a next second positioning portion 301, thereby achieving fine tuning.

The sentences that "the first positioning portion 102 forms a projection-receptacle engagement with the second positioning portion 301" means that one of them is provided with a protruded component, and the other is provided with a sunken component which may be a blind hole or a through hole, and the protruded component and the sunken component are mutually matched for positioning.

The rotating insert 100 is artificially or automatically positioned at the first position through a specialized positioning structure, such as a screw or a clamping structure, or may also be positioned through a pre-tightening force, such as an elastic member and the like.

Particularly, an elastic member is taken as an example. Referring to Fig. 2, the elastic member 500 is connected with the rotating insert 100, and a restoring force of the elastic member 500 always drives the rotating insert 100 to move toward the cover plate 300.

Further referring to Fig. 2, in order to maintain that the restoring force of the rotating insert 100 always drives the rotating insert 100 to move toward the cover plate 300, the rotating insert 100 has a protruded vertical end 106, the wheel body 200 has a wheel rotating hole 204, the vertical end 106 of the rotating insert 100 is inserted into the wheel rotating hole 204, and the elastic member 500 is arranged between a hole wall of the wheel rotating hole 204 and the vertical end 106, namely, may be arranged between a bottom wall of the wheel rotating hole 204 and a bottom wall of the vertical end 106, or may also be arranged at other positions.

The vertical end 106 can be of a cylinder shape, particularly, can be of a cylinder, a prism or a cylinder-like shape, while the shape of the wheel rotating hole 204 is matched with the vertical end 106 so as to ensure the vertical end 106 can freely rotate in the wheel rotating hole 204.

When a positioning column 102 is inserted into a positioning hole 301 of the cover plate 300, the elastic member 500 is in a natural state or a slightly compressed state, and the fine-tuning mechanism is in a locking state; when the positioning column 102 is disengaged from the positioning hole 301, the elastic member 500 is in a compressed state, and the fine-tuning mechanism is in an adjusted state.

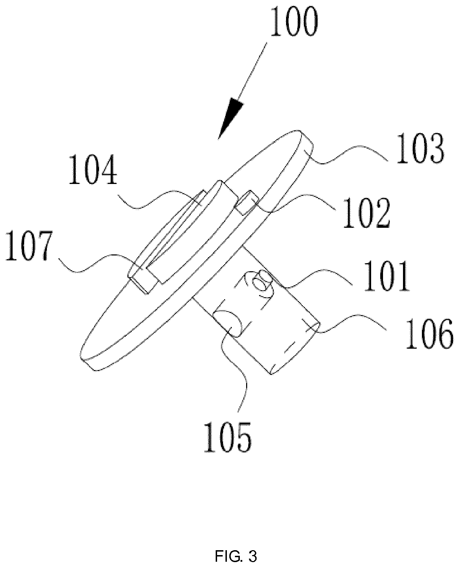

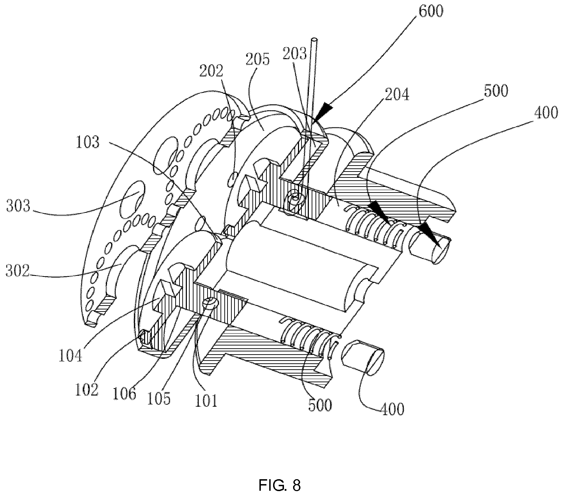

Referring Figs. 3 and 8, the mounting portion is used for fixing a traction line 600. Particularly, the mounting portion can be designed as being arranged in a mounting hole in the vertical end 106. Further, this mounting hole can include an insert round hole 101 and an insert counter bore 105, the insert round hole 101 is coaxial to the insert counter bore 105, and the diameter of the insert counter bore 105 is larger than that of the insert round hole 101. After passing through the insert round hole 101 of the rotating insert 100, the traction line 600 is fixed in the insert counter bore 105.

However, the wheel body 200 has a circle of gaps interacting with the wheel rotating hole 204. After the vertical end 106 is inserted into the wheel rotating hole 204, the traction line 6 extends from the gap, as shown Fig. 2.

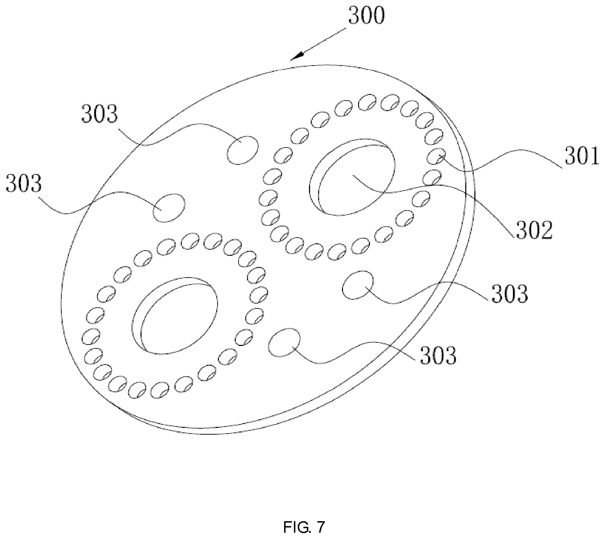

Further, referring to Figs.1, 2, 3 and 7, for conveniently adjusting the fine-tuning mechanism, the cover plate 300 has a cover plate through hole 302, the rotating insert 100 has a boss 104 for operating the rotation of the rotating insert 100, and the boss 104 is exposed from the cover plate through hole 302.

Furthermore, an end surface of the boss 104 may be provided with a groove 107 of a knife shape, a straight shape, a crisscross shape, a square shape or a hexagonal shape, so as to be matched with a common tool, such as a screwdriver, a square column or a hexagonal prism, and therefore the rotation of the rotating insert 100 is manipulated while pressing the rotating insert 100.

In addition, the external wall of the boss 104 can be made into shapes such as a hexagonal prism, a triangular prism or a rectangular pyramid. Similarly, the manipulation of the rotation of the rotating insert 100 is facilitated utilizing a tool.

The rotating insert 100 further includes an end disk 103, the vertical end 106 is arranged at one side of the end disk 103, the first positioning portion 102 is the positioning column (designated with 102 below), the second positioning portion 301 is the positioning hole (designated with 301), the positioning column 102 and the boss 104 are arranged at the other side of the end disk 103, as shown in Fig. 2, and the shape of the cross section of the rotating insert 100 is similar to a T shape.

Alternatively, in other examples, the first positioning portion may also be designed as the positioning hole, and the second positioning portion may be designed as the positioning column.

1-4 positioning columns 102 or more may be arranged, which are uniformly distributed along a circumference. The thicknesses of the positioning column 102 and the boss 104 are adaptive to the thickness of the cover plate 300, and are slightly larger than, slightly smaller than or equal to the thickness of the cover plate 300.

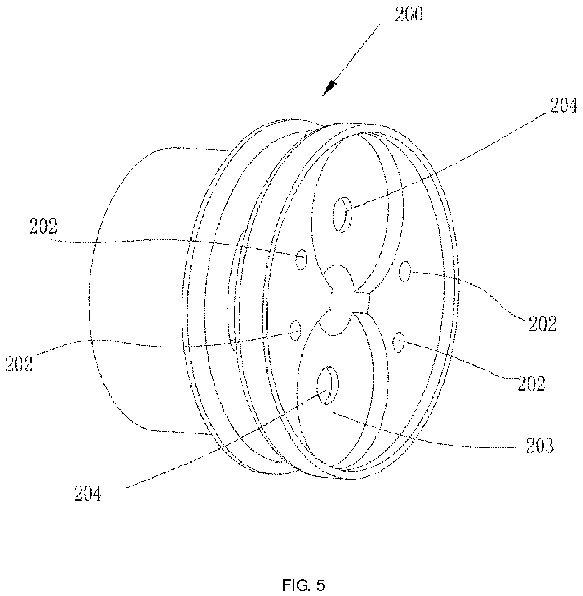

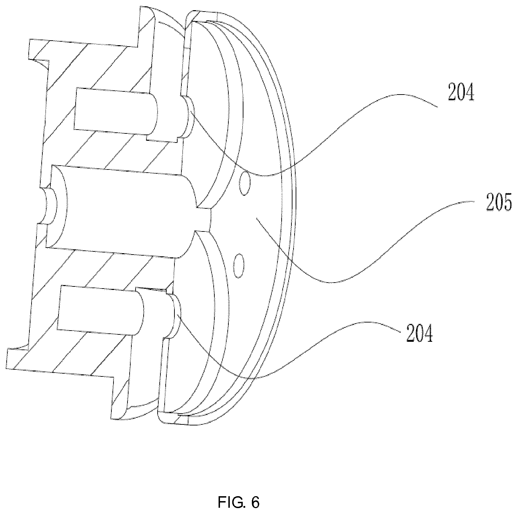

Referring to Figs. 5 and 6, the wheel body 200 has a cup-shaped body. The wheel rotating hole 204 is formed in a bottom wall 205 of the cup-shaped body, the cover plate 300 fixedly covers the cup-shaped body, and the rotating insert 100 is arranged in a cavity surrounded by a bottom wall 205 of the cup-shaped body and the cover plate 300.

The cover plate 300 can be a disk-shaped body, one end of the wheel body 200 is provided with a heavy tank whose deepness is adaptive to that of the cover plate 300, and the cover plate 300 is fixedly connected with the heavy tank.

Further, a bottom wall 205 of the cup-shaped body can be provided with a wheel counter bore 203 into which the end disk 103 of the rotating insert 100 is accommodated.

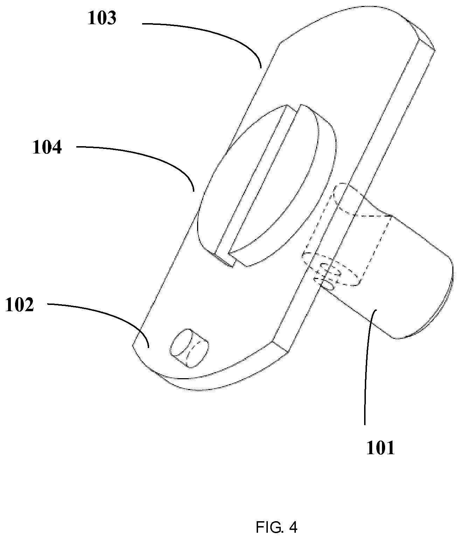

The end disk 103 of the rotating insert 100 may be a disk as shown in Fig. 3, or may be of a like-disk structure such as a kidney basin as shown in Fig. 4, as long as it is ensured that the rotating insert 100 can rotate in the wheel counter bore 203.

In this example, two rotating inserts 100 are arranged, and thus other corresponding structures are all represented as two groups. However, one or more inserts may be selected in other examples.

In a practical structure principle, the more the number (set as N) of the positioning holes 301 is, the higher adjustable precise is. The minimum regulation precise is set as Φ, and therefore, Φ=360/N, and the minimum fine tuning amount of the traction line 600 is L=π*r*Φ/180. The r is a distance from the center of the positioning hole 301 to the center of the cover plate through hole 302.

When the positioning column 102 is inserted into the positioning hole 301 in the cover plate 300, the elastic member 500 is in a natural state or a slightly compressed state, and the fine-tuning mechanism is in a locking state. When the positioning column 102 is disengaged from the positioning hole 301, the elastic member 500 is in a compressed state and is respectively abutted against the vertical end 106 and the bottom wall of the wheel rotating hole 204, and the fine-tuning mechanism is in an adjusted state.

Of course, as shown in Fig. 2, the wheel rotating hole 204 may be a blind hole integrated with the wheel body 200, and the bottom wall of the wheel rotating hole 204 is a bottom wall itself.

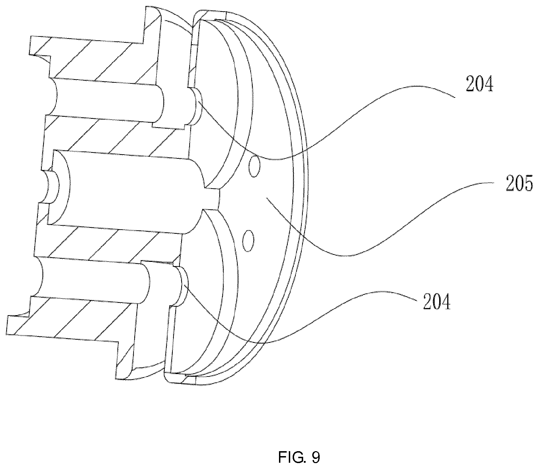

Alternatively, in some examples, in order to conveniently change the elastic member 500, as shown in Figs. 8 and 9, the wheel rotating hole 204 is formed by matching a threaded fastener 400 with the wheel body 200, and at this moment, the bottom wall of the wheel rotating hole 204 is a top end of the threaded fastener 400.

The threaded fastener 400 is a fixed bolt, which is meshed with the internal thread of the wheel rotating hole 204; the elastic member 500 is placed in the wheel rotating hole 204, one end of the elastic member 500 is abutted against the threaded fastener 400, and the other end of the elastic member 500 is abutted against with the vertical end 106.

In the process of use, an accessory tool is used to be inserted into a groove 107, and the rotating insert 100 is powerfully and downwardly pressed, so that the positioning column 102 and the boss 104 are respectively disengaged from the positioning hole 301 and the cover plate through hole 302, and meanwhile, the elastic member 500 is in a compressed state. By adjusting the rotation of the rotating insert 100, the traction line 600 rotates following the vertical end 106, and gradually stretches the traction line 600 until the state of the traction line 600 becomes a tight state from a loosen state. When the tight state is achieved, the accessory tool is withdrawn back, the rotating insert 100 is pulled outwardly under the effect of an elastic force of the elastic member 500, and the positioning column 102 and the boss 104 are re-inserted into the positioning hole 301 and the cover plate through hole 302, thereby completing the fine-tuning process.

Compared with the processing of the internal teeth and the external teeth in the prior art, the production difficulty of the present fine-tuning mechanism is reduced, and therefore cost is reduced. A sliding tool phenomenon occurs in the meshing process of the internal teeth and the external teeth in the prior art. According to the disclosure, the positioning column and the positioning hole are in plug type hole-hole matching, so the structure is precise and the stability is high, thereby avoiding the defect of sliding teeth. Meanwhile, in a fine-tuning link, the existing technology cannot quantitatively achieve fine tuning, and the number of teeth cannot be precisely controlled. In the disclosure, the positioning column moves under the through hole by rotating the rotating insert. When the fine-tuning requirement is achieved, the positioning column is inserted into the positioning hole so as to form hole-hole matching, thereby achieving quantitative fine tuning and precise angle control.

The disclosure is described with reference to particular examples as set forth above, and these examples are only for better understanding of the invention instead of limiting the invention. One of ordinary skill in the art can make variations or modifications to above embodiments under the teaching of this disclosure.

## Claims

1. An endoscope comprising a traction line, and a fine-tuning mechanism; wherein the fine-tuning mechanism comprises:
a rotating insert (100) comprising a first positioning portion (102) and a mounting portion for connecting the traction line (600);
a wheel body (200) on which the rotating insert (100) is mounted in a rotatable manner, wherein the rotating insert (100) is movable relative to the wheel body (200) along a rotation center line of the rotating insert (100); the rotating insert (100) is capable of being moved between a first position and a second postion; wherein the first position is configured for locking the rotating insert (100), and the second position is configured for unlocking the rotating insert (100); and the rotating insert (100) is capable of being positioned at the first position; and
a cover plate (300) fixed relative to the wheel body (200), wherein the rotating insert (100) is located between the cover plate (300) and the wheel body (200); the cover plate (300) comprises at least two second positioning portions (301) arranged around the rotation center line of the rotating insert (100); when the rotating insert (100) moves to the first position, the first positioning portion (102) forms a projection-receptacle engagement with one of the second positioning portions (301) which means that one of the first positioning portion (102) and second positioning portions (301) is provided with a protruded component, and the other is provided with a sunken component which is a blind hole or a through hole, and the protruded component and the sunken component are mutually matched for positioning; and
when the rotation insert (100) moves to the second position, the first positioning portion (102) is disengaged from the second positioning portion (301).

2. The endoscope of claim 1, wherein the fine-tuning mechanism further comprises an elastic member (500), wherein, the elastic member (500) is connected with the rotating insert (100), and a restoring force of the elastic member (500) always drives the rotating insert (100) to move toward the cover plate (300).

3. The endoscope of claim 2, wherein the rotating insert (100) has a projected vertical end (106), and the wheel body (200) has a wheel rotating hole (204) into which the vertical end (106) of the rotating insert (100) is inserted; and the elastic member (500) is arranged between a hole wall of the wheel rotating hole (204) and the vertical end (106).

4. The endoscope of claim 3, wherein the cover plate (300) has a cover plate through hole (302), and the rotating insert (100) has a boss (104) exposed from the cover plate through hole (302), for operating the rotation of the rotating insert (100).

5. The endoscope of claim 4, wherein an end surface of the boss (104) is provided with a groove of a knife shape, a straight shape, a Crisscross shape, a square shape or a hexagonal shape.

6. The endoscope of claim 4, wherein the rotating insert (100) comprises an end disk (103), the vertical end (106) is arranged at one side of the end disk (103), the first positioning portion (102) is a positioning column (102), the second positioning portion (301) is a positioning hole (301), and the positioning column (102) and the boss (104) are arranged at the other side of the end disk. (103)

7. The endoscope of claim 6, wherein the mounting portion is a mounting hole formed in the vertical end (106), the wheel body (200) has a circle of gaps crossed with the wheel rotating hole (204), and when the vertical end (106) is inserted into the wheel rotating hole (204), the traction line extends from the gap.

8. The endoscope of claim 6, wherein the wheel body (200) has a cup-shaped body, the wheel rotating hole (204) is formed in a bottom wall (205) of the cup-shaped body, the cover plate (300) fixedly covers the cup-shaped body, and the rotating insert (100) is arranged in a cavity surrounded by the bottom wall (205) of the cup-shaped body and the cover plate (300).

9. The endoscope of claim 8, wherein the bottom wall (205) of the cup-shaped body is provided with a wheel counter bore (203) into which the end disk (103) of the rotating insert (100) is accommodated.

10. The endoscope of claim 1, wherein two rotating inserts (100) are arranged.

## Patentansprüche

1. Endoskop mit einer Traktionslinie und einem Feinabstimmungsmechanismus, wobei der Feinabstimmungsmechanismus umfasst:
einen Dreheinsatz (100), der einen ersten Positionierungsabschnitt (102) und einen Befestigungsabschnitt zum Anschließen einer Traktionslinie (600) umfasst;
einen Radkörper (200), auf dem der Dreheinsatz (100) drehbar montiert ist, wobei der Dreheinsatz (100) relativ zu dem Radkörper (200) entlang einer Rotationsmittellinie des Dreheinsatzes (100) bewegbar ist; wobei der Dreheinsatz (100) in der Lage ist, zwischen einer ersten Position und einer zweiten Position zu bewegen; wobei die erste Position zum Verriegeln des Dreheinsatzes (100) konfiguriert ist und die zweite Position zum Entriegeln des Dreheinsatzes (100) konfiguriert ist, und wobei der Dreheinsatz (100) in der ersten Position positioniert werden kann; und
eine Abdeckplatte (300), die relativ zu dem Radkörper (200) befestigt ist, wobei sich der Dreheinsatz (100) zwischen der Abdeckplatte (300) und dem Radkörper (200) befindet; wobei die Abdeckplatte (300) mindestens zwei zweite Positionierungsabschnitte (301) umfasst, die um die Rotationsmittellinie des Dreheinsatzes (100) angeordnet sind;
wobei, wenn der Dreheinsatz (100) sich in die erste Position bewegt, der erste Positionierungsabschnitt (102) einen Vorsprung-Aufnahme-Eingriff mit einem der zweiten Positionierungsabschnitte (301) bildet, was bedeutet, dass einer von dem ersten Positionierungsabschnitt (102) und dem zweiten Positionierungsabschnitt (301) mit einem vorstehenden Bauteil versehen ist und der andere mit einem versenkten Bauteil versehen ist, das ein Sackloch oder ein Durchgangsloch ist, wobei das vorstehende Bauteil und das versenkte Bauteil zur Positionierung gegenseitig angepasst sind;
und wobei, wenn der Dreheinsatz (100) in die zweite Position bewegt, der erste Positionierungsabschnitt (102) vom zweiten Positionierungsabschnitt (301) gelöst wird.

2. Endoskop nach Anspruch 1, wobei der Feinabstimmungsmechanismus ferner ein elastisches Element (500) umfasst, wobei das elastische Element (500) mit dem Dreheinsatz (100) verbunden ist und eine Rückstellkraft des elastischen Elements (500) den Dreheinsatz (100) stets dazu antreibt, sich in Richtung der Deckplatte (300) zu bewegen.

3. Endoskop nach Anspruch 2, wobei der Dreheinsatz (100) ein vorstehendes vertikales Ende (106) umfasst und der Radkörper (200) einen Raddrehloch (204) umfasst, in den das vertikale Ende (106) des Dreheinsatzes (100) eingesetzt ist; und wobei das elastische Element (500) zwischen einer Lochwand des Raddrehlochs (204) und dem vertikalen Ende (106) angeordnet ist.

4. Endoskop nach Anspruch 3, wobei die Abdeckplatte (300) ein Abdeckplattendurchgangsloch (302) aufweist und der Dreheinsatz (100) einen Vorsprung (104) aufweist, der aus dem Abdeckplattendurchgangsloch (302) herausragt, um die Drehung des Dreheinsatzes (100) zu betätigen.

5. Endoskop nach Anspruch 4, wobei eine Endfläche des Vorsprungs (104) mit einer messerförmigen, einer geraden, einer kreuzförmigen, einer quadratischen oder einer sechseckigen Rille versehen ist.

6. Endoskop nach Anspruch 4, wobei der Dreheinsatz (100) eine Endscheibe (103) umfasst und das vertikale Ende (106) an einer Seite der Endscheibe (103) angeordnet ist, wobei der erste Positionierabschnitt (102) eine Positioniersäule (102) ist und der zweite Positionierabschnitt (301) ein Positionierloch (301) ist, und wobei die Positioniersäule (102) und der Vorsprung (104) an der anderen Seite der Endscheibe (103) angeordnet sind.

7. Endoskop nach Anspruch 6, wobei der Befestigungsabschnitt ein Befestigungsloch ist, das in dem vertikalen Ende (106) ausgebildet ist, wobei der Radkörper (200) einen Kreis von Lücken aufweist, die sich mit dem Raddrehloch (204) kreuzen, und wobei sich die Traktionslinie aus der Lücke erstreckt, wenn das vertikale Ende (106) in das Raddrehloch (204) eingesetzt ist.

8. Endoskop nach Anspruch 6, wobei der Radkörper (200) einen becherförmigen Körper aufweist, wobei das Raddrehloch (204) in einer Bodenwand (205) des becherförmigen Körpers ausgebildet ist, wobei die Abdeckplatte (300) den becherförmigen Körper fest abdeckt und der Dreheinsatz (100) in einem von der Bodenwand (205) des becherförmigen Körpers und der Abdeckplatte (300) umgebenen Hohlraum angeordnet ist.

9. Endoskop nach Anspruch 8, wobei die Bodenwand (205) des becherförmigen Körpers mit einer Radsenkbohrung (203) versehen ist, in der die Endscheibe (103) des Dreheinsatzes (100) aufgenommen ist.

10. Endoskop nach Anspruch 1, wobei zwei Dreheinsätze (100) angeordnet sind.

## Revendications

1. Endoscope comprenant une ligne de traction et un mécanisme de réglage fin ; dans lequel le mécanisme de réglage fin comprend :
Un insert rotatif (100) comprenant une première partie de positionnement (102) et une partie de montage pour connecter la ligne de traction (600) ;
Un corps de roue (200) sur lequel l'insert rotatif (100) est monté avec faculté de rotation, **caractérisé en ce que** : l'insert rotatif (100) est mobile par rapport au corps de roue (200) le long d'une ligne centrale de rotation dudit insert rotatif (100) ; l'insert rotatif (100) est capable de se déplacer entre une première position et une seconde position dans lesquelles la première position est configurée pour verrouiller l'insert rotatif (100), alors que la seconde position est configurée pour déverrouiller l'insert rotatif (100) ; et l'insert rotatif (100) peut être positionné à la première position ; et
Une plaque couvercle (300) fixe par rapport audit corps de roue (200), **caractérisé en ce que** : l'insert rotatif (100) est positionné entre la plaque couvercle (300) et le corps de roue (200) ; la plaque couvercle (300) comprend au moins deux secondes parties de positionnement (301) disposées autour de la ligne centrale de rotation de l'insert rotatif (100) ; lorsque l'insert rotatif (100) se déplace vers une première position, la première partie de positionnement (102) permet une solidarisation de type projection-réceptacle avec l'une des secondes parties de positionnement, ce qui signifie que : une parmi la première partie de positionnement (102) et les seconde parties de positionnement (301) est pourvue d'un composant en saillie, l'autre est pourvue d'un composant creux qui est un trou borgne ou traversant, et ledit composant en saillie et ledit composant creux sont mutuellement adaptés pour réaliser le positionnement ; lorsque l'insert rotatif (100) se déplace vers une seconde position, la première partie de positionnement (102) se désolidarise de ladite seconde partie de positionnement (301).

2. Endoscope selon la revendication 1, **caractérisé en ce que** : le mécanisme de réglage fin en outre un élément élastique (500) ; ledit élément élastique (500) est relié à l'insert rotatif (100) ; et une force de rappel de l'élément élastique (500) entraîne toujours l'insert rotatif (100) pour se déplacer vers la plaque couvercle (300).

3. Endoscope selon la revendication 2, **caractérisé en ce que** : l'insert rotatif (100) a une extrémité verticale en saillie (106), et le corps de roue (200) présente un trou de rotation de roue (204) dans lequel l'extrémité verticale (106) de l'insert rotatif (100) est insérée ; d'ailleurs, l'élément élastique (500) est disposé entre une paroi du trou de rotation de roue (204) et l'extrémité verticale (106).

4. Endoscope selon la revendication 3, **caractérisé en ce que** : la plaque couvercle (300) présente un trou traversant (302), et l'insert rotatif (100) a un bossage (104) exposé depuis la plaque couvercle à travers le trou (302), pour réaliser la rotation de l'insert rotatif (100).

5. Endoscope selon la revendication 4, **caractérisé en ce qu'**une surface d'extrémité dudit bossage (104) est pourvue d'une rainure en forme de couteau / droite / entrecroisée / carrée / hexagonale.

6. Endoscope selon la revendication 4, **caractérisé en ce que** : l'insert rotatif (100) comprend un disque d'extrémité (103) ; ladite extrémité verticale (106) est disposée sur un côté dudit disque d'extrémité (103) ; ladite première partie de positionnement (102) est une colonne (102), alors que ladite seconde partie de positionnement (301) est un trou (301) ; la colonne de positionnement (102) et le bossage (104) sont disposés sur l'autre côté dudit disque d'extrémité (103).

7. Endoscope selon la revendication 6, **caractérisé en ce que** : ladite partie de montage est un trou de montage formé dans l'extrémité verticale (106) ; ledit corps de roue (200) présente un cercle d'espaces croisé avec ledit trou de rotation de roue (204) ; lorsque l'extrémité verticale (106) est insérée dans ledit trou de rotation de roue (204), la ligne de traction s'étend depuis l'espace.

8. Endoscope selon la revendication 6, **caractérisé en ce que** : ledit corps de roue (200) est en forme de coupe ; ledit trou de rotation de roue (204) est formé dans une paroi inférieure (205) dudit corps en forme de coupe ; ladite plaque couvercle (300) recouvre de manière fixe ledit corps en forme de coupe ; et l'insert rotatif (100) est disposé dans une cavité entourée par ladite paroi inférieure (205) dudit corps en forme de coupe et par ladite plaque couvercle (300).

9. Endoscope selon la revendication 8, **caractérisé en ce que** ladite paroi inférieure (205) dudit corps en forme de coupe est pourvue d'un contre-alésage de roue (203) qui loge ledit disque d'extrémité (103) de l'insert rotatif (100).

10. Endoscope selon la revendication 1, **caractérisé en ce que** lesdits deux inserts rotatifs (100) sont disposés.
